(19) Europäisches Patentamt
European Patent Office
Office européen des brevets

(11) Veröffentlichungsnummer: **0 415 189 A2**

# (12) EUROPÄISCHE PATENTANMELDUNG

(21) Anmeldenummer: **90115683.6**

(22) Anmeldetag: **16.08.90**

(51) Int. Cl.5: **C07C 213/00**, C25B 3/04

(30) Priorität: **26.08.89 DE 3928290**

(43) Veröffentlichungstag der Anmeldung:
**06.03.91 Patentblatt 91/10**

(84) Benannte Vertragsstaaten:
**BE CH DE ES FR GB IT LI NL**

(71) Anmelder: **BASF Aktiengesellschaft**
**Carl-Bosch-Strasse 38**
**W-6700 Ludwigshafen(DE)**

(72) Erfinder: **Steiniger, Michael, Dr.**
**Wolfskeule 12**
**W-6730 Neustadt(DE)**
Erfinder: **Puetter, Hermannn, Dr.**
**Haardter Strasse 1**
**W-6730 Neustadt(DE)**
Erfinder: **Grosse Brinkhaus, Karl-Heinz, Dr.**
**Kaiserslauterer Strasse 112**
**W-6702 Bad Duerkheim(DE)**

## (54) Verfahren zur Herstellung von Aminobenzylalkoholen.

(57) Verfahren zur Herstellung von Aminobenzylalkoholen der Formel

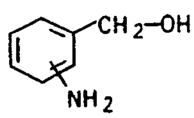

I,

bei dem man Aminobenzoesäureester der Formel

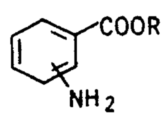

II,

in der R einen Alkylrest mit 1 - 4 C-Atomen bedeutet, in einem stark sauren Elektrolyten kathodisch reduziert.

EP 0 415 189 A2

## VERFAHREN ZUR HERSTELLUNG VON AMINOBENZYLALKOHOLEN

Diese Erfindung betrifft ein Verfahren zur Herstellung von Aminobenzylalkoholen durch elektrochemische Reduktion von Aminobenzoesäureester.

Es sind schon zahlreiche Verfahren zur Reduktion aromatischer Carbonsäuren oder deren Ester vorgeschlagen worden. So hat man für die Herstellung von o-Aminobenzylalkohol aus Anthranilsäurederivaten z.B. komplexe Hydride herangezogen. Neben Lithiumaluminiumhydrid (J. Am. Chem. Soc. 69, 2548 (1947)) sind beispielsweise auch Boran-Methylsulfid (J. Org. Chem. 39, 3052 (1974)) oder Lithiumtriethylborhydrid (Aldrichimica Acta 7, 32 (1974)) geeignet. Diese komplexen Hydride haben jedoch den Nachteil, daß sie teuer, sauerstoff- und/oder feuchtigkeitsempfindlich sind und oft nur in trocknen, aprotischen Lösungsmitteln unter besonderen Vorsichtsmaßnahmen umgesetzt werden können.

Aus J. Chem. Soc. 1942, 98-102 ist die elektrochemische Reduktion von Anthranilsäure zu o-Aminobenzylalkohol bekannt. Dieses Verfahren hat jedoch den Nachteil, daß es nur in mäßigen Ausbeuten zu einem Produkt führt, daß erst nach einer verlustreichen Umkristallisation in reiner Form isoliert werden kann.

Man hat auch schon Ester der Benzoesäure elektrochemisch reduziert. So wird in M.M. Baizer und H. Lund: "Organic Elelctrochemistry", M. Dekker, New York 1983, S. 380-381 angegeben, daß bei der kathodischen Reaktion von Methyl- oder Ethylbenzoat in stark saurem Medium an einer Bleikathode ein Gemisch entsteht, das annähernd zu gleichen Teilen aus Benzylalkohol und Benzylether besteht. Auch die Elektroreduktion von stickstoffhaltigen heterocyclisch-aromatischen Carbonsäureestern, wie Imidazol-4-carbonsäureester im stark sauren wäßrigen Elektrolyten führt zu einem 1:1-Gemisch aus Alkohol und Ether (US-PS 4 055 573). Die Bildung derartiger Gemische wird durch folgenden Reaktionsmechanismus erklärt (s. die oben erwähnten Literaturstelle "Organic Electrochemistry", S. 380).

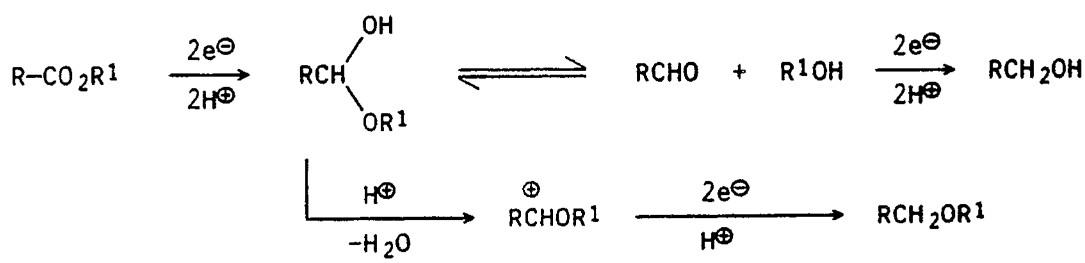


Es hat daher nicht an Versuchen gefehlt, die Bildung der Benzylalkohole zur Hauptreaktion zu machen. Das gelingt dann, wenn man Benzoesäureester in einem sauren wäßrigen Elektrolyten, der wasserlösliche Hilfsstoffe, wie Alkohole, Tetrahydrofuran oder Acetonitril enthält, bei einem pH-Wert bis 4 (J 6 2297 482) oder in alkoholischer Lösung in Gegenwart von Tetraalkylammoniumsalzen und Essigsäure bei einem pH von über 3 (Liebigs Ann. Chem. 1977, 2036-2047; DE 2 428 878) elektroreduziert.

Von einer Übertragung dieser Methoden auf eine elektrochemische Herstellung von Aminobenzylalkoholen aus Anthranilsäureestern konnten keine brauchbaren Ergebnisse erwartet werden. Es war vielmehr damit zu rechnen, daß es durch Nebenreaktionen zwischen der Aminogruppe der Ausgangsverbindung und der Aldehydgruppe der bei der Reduktion zwangsläufig entstehenden Zwischenverbindung in erheblichem Maße zu einer Bildung dimerer bzw. oligomerer Produkte kommt und damit nur stark verminderte Ausbeuten erhalten werden.

Es wurde nur gefunden, daß man Aminobenzylalkohole der Formel

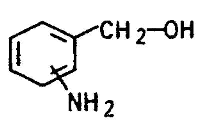

I,

vorteilhaft herstellen kann, wenn man Aminobenzoesäureester der Formel

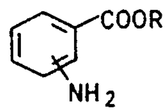

II,

in der R einen Alkylrest mit 1 bis 4 C-Atomen bedeutet, in einem stark sauren Elektrolyten kathodisch reduziert. Nach dem neuen Verfahren erhält man die Aminobenzylalkohole überraschenderweise in guten Ausbeuten und hoher Reinheit, ohne daß es zu einer nennenswerten Bildung von Aminobenzylalkylethern kommt.

In den Aminobenzoesäureestern der Formel (I) steht die Aminogruppe in ortho-, meta- oder para-Stellung. R steht für einen Alkylrest mit 1 bis 4 C-Atomen, wie für Methyl, Ethyl, Propyl oder Butyl. Als Ausgangsverbindungen sind die Methylester, insbesondere der Anthranilsäuremethylester bevorzugt.

Für die elektrochemische Reduktion wird der Aminobenzoesäureester in gelöster oder suspendierter Form, vorzugsweise in einem wäßrigen Elektrolyten bei einem pH-Wert von kleiner als 1 eingesetzt. Die Einstellung des pH-Wertes kann durch zugabe von Säuren, wie Salzsäure, Phosphorsäure, Benzolsulfonsäure, p-Toluolsulfonsäure oder vorzugsweise Schwefelsäure erfolgen.

Bevorzugt werden wäßrige saure Katholyten eingesetzt, die keine Elektrolyse-Hilfsstoffe enthalten. Ihr Gehalt an Benzoesäureester liegt z.B. zwischen 1 und 10 Gew.%, vorzugsweise zwischen 2 und 7 Gew.%. So hat der Katholyt z.B. einen Gehalt an Benzoesäureester von 2 bis 10 Gew.% an Schwefelsäure von 5 bis 20 Gew.% und an Wasser von 70 bis 93 Gew.%. Als Anolyt verwendet man zweckmäßigerweise wäßrige Mineralsäuren, vorzugsweise wäßrige Schwefelsäure.

Die Elektrolyse wird in üblichen Elektrolysezellen (Trogzellen, Durchflußzellen usw.), vorzugsweise in geteilten zellen, durchgeführt. Besonders geeignet sind monopolare oder bipolare zellen, bei denen Anoden- und Kathodenraum durch ein Diaphragma, z. B. aus einer porösen Platte, einem Glasfilter oder einer handelsüblichen Ionenaustauschermembrane, getrennt sind. Zur Aufrechterhaltung einer ausgeglichenen pH-Bilanz werden bevorzugt Kationenaustauschermembranen als Diaphragma eingesetzt.

Man verwendet zweckmäßigerweise Kathoden mit einer hohen Wasserstoffüberspannung, wie solche aus Quecksilber, Cadmium, Blei, Zink, Zinn, Graphit oder amalgierten Metallen (Pb, Cu). Bevorzugt wird Blei als Kathodenmaterial eingesetzt. Um ein Absinken der Stromausbeute zu vermeiden, kann es nach längeren Elektrolysezeiten zweckmäßig sein, die Kathode durch kurzzeitiges Kurzschließen zu reinigen. Die Wahl des Anodenmaterials spielt keine erfindungswesentliche Rolle. Zweckmäßigerweise wird man solche Materialien verwenden, die unter anodischer Belastung weitgehend stabil sind, wie Edelmetalle, mit Edelmetalloxiden dotiertes Titan, Mangandioxid, Graphit, bevorzugt jedoch Bleidioxid.

Man führt die Elektrolyse z. B. bei Temperaturen zwischen 0 und 50 °C, vorzugsweise bei 10 bis 30 °C durch. Die Kathodenstromdichte beträgt z. B. 1 bis 30 $A/dm^2$, insbesondere 3 bis 15 $A/dm^2$.

Nach der Elektrolyse arbeitet man z. B. so auf, daß man den Elektrolyseaustrag mit einer Base wie Natronlauge oder vorzugsweise Ammoniak alkalisch stellt (pH 8-10), die ausgefallenen Oligomeren durch Filtration entfernt und die Mutterlauge mit Methyl-tert.Butylether extrahiert. Nach dem Entfernen des Extraktionsmittels erhält man z.B. aus Anthranilsäuremethylester elfenbeinweißen o-Aminobenzylalkohol in 95 %iger Reinheit, so daß sich weitere Reinigungsschritte, wie eine Umkristallisation erübrigen.

Die Aminobenzylalkohole sind wertvolle Zwischenprodukte, z.B. für die Herstellung von Pharmazeutika.

## Beispiele

### Beispiel 1

Ein Katholyt bestehend aus 90 g Anthranilsäuremethylester, 200 g konzentrierter Schwefelsäure und 1710 g Wasser (pH des Katholyten = 0,2) wird mit einer Temperatur von 20 °C über ein Vorratsgefäß und Wärmetauscher durch den Kathodenraum einer geteilten Elektrolysezelle gepumpt. Der Kathodenraum mit einer Bleikathode (Fläche 2 $dm^2$) wurde vom Anodenraum mit einer $Pb/PbO_2$-Anode (Fläche 2 $dm^2$) durch eine im Handel unter der Bezeichnung ®NAFION erhältliche Kationenaustauchermembrane getrennt. Der Anolyt besteht aus einer 10 %igen wäßrigen Schwefelsäure. Die Elektrolyse wird mit einer Stromdichte von 7,5 $A/dm^2$ betrieben. Nach 12 Stunden Elektrolysedauer (180 Ah = 10 F/mol) wird die Elektrolyse abgebrochen und der Katholyt aufgearbeitet.

Der orangegefärbte Elektrolyseaustrag wird mit wäßriger Ammoniaklösung auf einen pH-Wert von 9 bis 10 gestellt. Der ausgefallene Niederschlag wird über eine Drucknutsche abgesaugt. Nach dem Trocknen

des Filterrückstandes erhält man 10,3 g eines Oligomeren. Die alkalische Mutterlauge wird viermal mit 500 ml Methyl-tert.Butylether extrahiert und am Rotationsverdampfer scharf eingeengt. Man erhält 52,8 g elfenbeinweißen o-Aminobenzylalkohol in > 95 %iger Reinheit (Fp. 81-83 °C, Lit.: 83 °C) entsprechend einer Materialausbeute von 72 %. Durch eine gaschromatographische Analyse (30 m Kapillarsäule DB 1701) des Produktes wird ein Gehalt an o-Aminobenzylmethylether von kleiner als 1 % festgestellt.

Beispiel 2

Ein Katholyt bestehend aus 140 g Anthranilsäuremethylester, 300 g konz. Schwefelsäure und 1660 g Wasser (pH des Katholyten = -0,3) wird analog zu Beispiel 1 bei einer Stromdichte von 10 $A/dm^2$ elektrolysiert. Nach einem Ladungsverbrauch entsprechend 10 F/mol Anthranilsäuremethylester wird die Elektrolyse abgebrochen und, wie in Beispiel 1 beschrieben, aufgearbeitet. Man erhält 80,3 g o-Aminobenzylalkohol in > 95 %iger Reinheit (Fp. 81-82 °C) entsprechend einer Materialausbeute von 70 %.

Beispiel 3

Ein Katholyt bestehend aus 90 g m-Aminobenzoesäureethylester, 200 g konzentrierter Schwefelsäure und 1710 g Wasser (pH des Katholyten = 0,18) wird entsprechend Beispiel 1 elektrolysiert und aufgearbeitet (Extraktionsmittel Isobutylacetat). Man erhält 39,4 g m-Aminobenzylalkohol in > 95 %iger Reinheit (Fp. 88-90 °C, Lit. 90-92 °C) entsprechend einer Materialausbeute von 43 %.

## Ansprüche

1. Verfahren zur Herstellung von Aminobenzylalkoholen der Formel

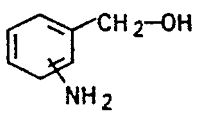

I,

dadurch gekennzeichnet, daß man Aminobenzoesäureester der Formel

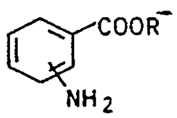

II,

in der R einen Alkylrest mit 1 - 4 C-Atomen bedeutet, in einem stark sauren Elektrolyten kathodisch reduziert.

2. Verfahren nach Anspruch 1, dadurch gekennzeichnet, daß man die Reduktion in einem Wasser enthaltenden Elektrolyten bei einem pH-Wert von kleiner als 1 durchführt.

3. Verfahren nach Anspruch 1, dadurch gekennzeichnet, daß man als Aminobenzoesäureester Anthranilsäureester einsetzt.

4. Verfahren nach den Ansprüchen 1 und 3, dadurch gekennzeichnet, daß man die elektrochemische Reduktion in einer geteilten Elektrolysezelle durchführt, wobei man einen Katholyten mit einem Gehalt an Aminobenzoesäureester von 2 bis 10 Gew.%, an Schwefelsäure von 5 bis 20 Gew.% und an Wasser von 70 bis 93 Gew.% verwendet.